# EUROPEAN PATENT APPLICATION

(11) **EP 3 520 803 A1**
(43) Date of publication of application: **07.08.2019**
(21) Application number: 18154553.4
(22) Date of filing: 31.01.2018
(51) Int. Cl.: A61K 38/13, A61K 31/436, A61K 31/165, A61K 31/19, A61K 31/365, A61K 31/4706, A61K 31/519, A61K 31/5513, A61K 31/573, A61K 31/675, A61P 25/00, A61P 37/00

(54) **NOVEL USES**

(71) Applicant: Zarodex Therapeutics Limited, Oxford, Oxfordshire OX3 0DW (GB)
(72) Inventor: The designation of the inventor has not yet been filed
(74) Representative: Teuten, Andrew John

(57) **Abstract**

This invention relates to the compound, clozapine, and its major active metabolite, norclozapine, and prodrugs thereof and pharmaceutically acceptable salts and solvates thereof for use in the treatment or prevention of a pathogenic immunoglobulin driven B cell disease. The invention also provides pharmaceutical compositions containing such compounds.

## Description

### Technical Field

This invention relates to a compound and pharmaceutical compositions containing such compound for use in the treatment or prevention of a pathogenic immunoglobulin driven B cell disease.

### Background to the invention

The compound associated with this invention is known as clozapine i.e. the compound of the following structure:

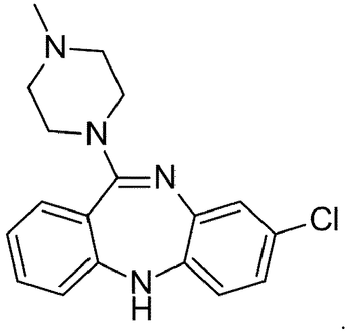

Clozapine has a major active metabolite known as norclozapine (Guitton C. et al., 1999) which has the following structure:

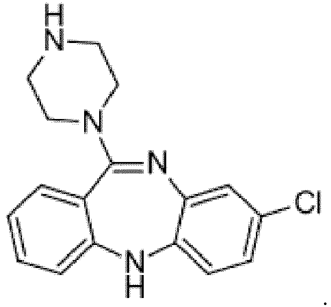

Clozapine is known as a treatment for resistant schizophrenia. Schizophrenia is an enduring major psychiatric disorder affecting around 1% of the population. Apart from the debilitating psychiatric symptoms it has serious psychosocial consequences with an unemployment rate of 80-90% and a life expectancy reduced by 10-20 years. The rate of suicide among people with schizophrenia is much higher than in the general population and approximately 5% of those diagnosed with schizophrenia commit suicide.

Clozapine is an important therapeutic agent and is included on the WHO list of essential medicines. It is a dibenzo-diazepine atypical antipsychotic, and since 1990 the only licensed therapy in the UK for the 30% of patients with treatment-resistant schizophrenia (TRS). It shows superior efficacy in reducing both positive and negative symptoms in schizophrenic patients and is effective in approximately 60% of previously treatment refractive patients with a significant reduction in suicide risk. The National Institute for Health and Clinical Excellence (NICE) guideline recommends adults with schizophrenia which has not responded adequately to treatment with at least 2 antipsychotic drugs (at least one of which should be a non-clozapine second generation antipsychotic) should be offered clozapine.

Clozapine is associated with serious adverse effects including seizures, intestinal obstruction, diabetes, thromboembolism, cardiomyopathy and sudden cardiac death. It can also cause agranulocytosis (cumulative incidence 0.8%); necessitating intensive centralised registry based monitoring systems to support its safe use. In the UK there are three electronic registries (www.clozaril.co.uk, www.denzapine.co.uk and www.ztas.co.uk) one for each of the clozapine suppliers. Mandatory blood testing is required weekly for the first 18 weeks, then every two weeks from weeks 19-52 and thereafter monthly with a 'red flag' cut-off value for absolute neutrophil count (ANC) of less than 1500/µL for treatment interruption.

In 2015, the Federal Drug Administration (FDA) merged and replaced the six existing clozapine registries in the United States combining data from over 50,000 prescribers, 28,000 pharmacies and 90,000 patients records into a single shared registry for all clozapine products, the Clozapine Risk Evaluation and Mitigation Strategy (REMS) Program (www.clozapinerems.com). Changes were introduced lowering the absolute neutrophil count (ANC) threshold to interrupt clozapine treatment at less than 1000/µL in general, and at less than 500/µL in benign ethnic neutropenia (BEN). Prescribers have greater flexibility to make patient-specific decisions about continuing or resuming treatment in patients who develop moderate to severe neutropenia, and so maximize patient benefit from access to clozapine.

Schizophrenia is associated with a 3.5 fold increased chance of early death compared to the general population. This is often due to physical illness, in particular chronic obstructive pulmonary disease (COPD) (Standardised Mortality Ratio (SMR) 9.9), influenza and pneumonia (SMR 7.0). Although clozapine reduces overall mortality in severe schizophrenia, there is a growing body of evidence linking clozapine with elevated rates of pneumonia-related admission and mortality. In an analysis of 33,024 patients with schizophrenia, the association between second generation antipsychotic medications and risk of pneumonia requiring hospitalization was highest for clozapine with an adjusted risk ratio of 3.18 with a further significant increase in risk associated with dual antipsychotic use (Kuo CJ. et al., 2013). Although quetiapine, olanzapine, zotepine, and risperidone were associated with a modestly increased risk, there was no clear dose-dependent relationship and the risk was not significant at time points beyond 30 days (Leung JG. et al., 2017 and Stoeker ZR. et al., 2017).

In a 12 year study of patients taking clozapine, 104 patients had 248 hospital admissions during the study period. The predominant admission types were for treatment of either pulmonary (32.2%) or gastrointestinal (19.8%) illnesses. The commonest pulmonary diagnosis was pneumonia, (58% of pulmonary admissions) and these admissions were unrelated to boxed warnings (Leung JG. et al., 2016).

In a further nested case control study clozapine was found to be the only antipsychotic with a clear dose-dependent risk for recurrent pneumonia, this risk increased on re-exposure to clozapine (Hung GC. et al., 2016).

While these studies underscore the increased admissions or deaths from pneumonia and sepsis in patients taking clozapine over other antipsychotics, the focus on extreme outcomes (death and pneumonia) may underestimate the burden of less severe but more frequent infections such as sinusitis, skin, eye, ear or throat infections and community acquired and treated pneumonia. Infection may represent an important additional factor in destabilizing schizophrenia control and clozapine levels.

Various mechanisms for the increase in pneumonia have been suggested, including aspiration, sialorrhoea and impairment of swallowing function with oesophageal dilatation, hypomotility and agranulocytosis. In addition, cigarette smoking is highly prevalent among patients with schizophrenia as a whole and represents an independent risk factor for pneumonia incidence and severity.

A small amount of research into the immunomodulatory properties of clozapine has been performed:

Hinze-Selch et al (1998) describes clozapine as an atypical antipsychotic agent with immunomodulatory properties. This paper reports that patients that received clozapine treatment for six weeks showed significant increases in the serum concentrations of IgG, but no significant effect was found on IgA or IgM concentrations or on the pattern of autoantibodies.

Jolles et al (2014) reports studies on the parameter "calculated globulin (CG)" as a screening test for antibody deficiency. Patients with a wide range of backgrounds were selected from thirteen laboratories across Wales. Of the patients with significant antibody deficiency (IgG <4g/L, reference range 6-16g/L), identified on CG screening from primary care, clozapine use was mentioned on the request form in 13% of the samples. However, antibody deficiency is not a listed side effect of clozapine in the British National Formulary (BNF), nor does antibody testing constitute part of current clozapine monitoring protocols.

Another study by Lozano et al., (2016) reported an overall decrease of mean plasma levels of IgM in the study group (which consisted of psychiatric outpatients who took clozapine for at least five years) compared to the control group, and also reported that no differences were found between the groups with respect to IgA, IgG, absolute neutrophil count and white blood cell count.

Consequently, given these mixed results that have been reported, the immunomodulatory properties of clozapine and its effect on immunoglobin levels are neither clear nor understood in the art.

Pathogenic immunoglobulin (including IgG, IgA and IgM) driven diseases result from secretion of autoantibodies (principally IgG and/or IgA) by antibody secreting cells ("ASCs", collectively plasmablasts and plasma cells, these being types of mature B cell). These antibodies target a variety of self-antigens which have been characterised in many of these conditions. There is rarely an increase in overall immunoglobulins as the pathological process is driven by the secretion of specific immunoglobulins which constitute a small percentage of the total immunoglobulins. Secretion of IgG antibodies and IgA antibodies are from ASCs, and ASCs are generated secondary to the differentiation of class-switched and unswitched memory B cells, these being further types of mature B cell. Various lines of evidence suggest this is a highly-dynamic process, with ongoing differentiation occurring almost constantly.

CD19(+) B cells and CD19(-) B plasma cells are drivers of pathogenic immunoglobulin driven B cell diseases. In particular, pathogenic IgG and IgA driven B cell diseases represent a substantial proportion of all autoimmune diseases. The most prominent, but not the sole mechanism through which pathogenic immunoglobulin driven B cells cause disease, is through auto-antibody production. Established pathogenic IgG immunoglobulin diseases include Pemphigus and Pemphigoid. Pemphigus, which has been designated to be an orphan disease, is an autoimmune interepithelial blistering disease characterised by loss of normal cell-cell adhesion (acantholysis). The antibodies involved are against desmoglein 3. If left untreated, it can be fatal, usually from overwhelming opportunistic infection due to loss of skin barrier function and from electrolyte loss. Pemphigoid is characterised by the formation of blister at the space between the epidermis and dermis skin layers. The antibodies involved are against dystonin and/or type XVII collagen.

Pathogenic immunoglobulin driven B cell diseases are poorly treated and as a result they have substantial mortality and morbidity rates, even for the "benign" diseases. Certain current advanced therapies are directed at mature B cells. For example, belimumab is a human monoclonal antibody that inhibits B cell activating factor. Atacicept is a recombinant fusion protein that also inhibits B cell activating factor. However, memory B cells may be resistant to therapies such as belimumab or atacicept which target survival signals such as B cell activation factor (Stohl et al, 2012). The importance of memory B cells in the pathogenesis of autoimmune disorders was also demonstrated by the lack of efficacy of atacicept in treating rheumatoid arthritis and multiple sclerosis (Richez et al., 2014; Kappos et al., 2014). Plasmapheresis and immunoabsorption involve the removal of disease-causing autoantibodies from the patient's bloodstream. However, these treatments have limited efficacy or are complex and costly to deliver. CAR-T methods directed at CD19(+) B cells leaves CD19(-) B plasma cells intact, which makes it ineffective.

Rituximab is a drug that is currently used to treat some pathogenic IgG driven B cell diseases. It targets B cells that express CD20. However, CD20 is only expressed on a limited subset of B cells. It also does not target plasma cells. This limited expression of CD20 and lack of effect on plasma cells explains the limited efficacy of rituximab in a variety of diseases, both benign and malignant, despite being definitively of B cell origin. Rituximab does not appear to have any effect on IgA-secreting plasmablasts/plasma cells, and consequently the associated IgA driven B cell diseases (Yong et al., 2015).

Thus, there is a major unmet medical need for new treatments against pathogenic immunoglobulin driven B cell diseases.

### Summary of the invention

It has been found by the inventors that clozapine has a potential important therapeutic effect as it significantly reduces class switched memory B cells ("CSMB"), a type of mature B cell. This will consequently reduce the numbers of ASCs, and hence the secretion of specific immunoglobulins including the pathogenic immunoglobulins. Clozapine was also observed to cause a reduction in levels of plasmablasts, another type of mature B cell. This functional effect on persistent and long lived adaptive B cell and plasma cell function may ameliorate the diseases driven by the persistent generation of pathogenic immunoglobulins that drives the pathology of pathogenic immunoglobulin driven B cell diseases. The inventors' new data demonstrates a very significant effect on the number of circulating class switched memory B cells, an effect on the number of plasmablasts and importantly, through the lack of recall response to common vaccines, an effect on the function of the class switched memory B cells and plasmablasts resulting in specific reduction of antibodies targeting a previously exposed antigen. The inventors' new data also demonstrates an effect of the drug in reducing total IgG, IgA and IgM levels after administration. With the lack of effect on other B cells, shown by the lack of depletion of other sub-types and total B cell numbers but with a particular reduction in CSMBs and plasmablasts, this observation strongly supports a functional effect on CSMBs and plasmablasts which are central to long lived production of pathogenic antibodies in pathogenic immunoglobulin (particularly IgG and IgA) driven B cell diseases.

Thus, the present invention provides a compound selected from clozapine, norclozapine and prodrugs thereof and pharmaceutically acceptable salts and solvates thereof for use in the treatment or prevention of a pathogenic immunoglobulin driven B cell disease in a subject, in particular, wherein said compound causes mature B cells to be inhibited in said subject.

### Brief Description of the Drawings

**Figures 1A-C.** show the relative frequencies of numbers of patients at each serum concentration value for IgG, IgA and IgM respectively for clozapine-treated patients (black) and clozapine-naïve patients (grey).
**Figure 2****.** shows the effect of duration of clozapine use on serum IgG levels.
**Figure 3****.** shows the number of class switched memory B cells (CSMB) (CD27+/IgM-/IgD-, expressed as a percentage of total CD19+ cells) in healthy controls, in patients taking clozapine referred to clinic and in patients with common variable immunodeficiency disorder (CVID).
**Figure 4****.** shows the number of plasmablasts (CD38+++/IgM-, expressed as a percentage of total CD19+ cells) in healthy controls, in patients taking clozapine referred to clinic and in patients with common variable immunodeficiency disorder (CVID).

### Detailed description of the invention

The present invention also provides a method of treatment or prevention of a pathogenic immunoglobulin driven B cell disease in a subject by administering to said subject an effective amount of a compound selected from clozapine, norclozapine and prodrugs thereof and pharmaceutically acceptable salts and solvates thereof, in particular, wherein said compound causes mature B cells to be inhibited in said subject.

The present invention also provides use of a compound selected from clozapine, norclozapine and prodrugs thereof and pharmaceutically acceptable salts and solvates thereof in the manufacture of a medicament for the treatment or prevention of a pathogenic immunoglobulin driven B cell disease in a subject, in particular, wherein said compound causes mature B cells to be inhibited in said subject.

Clozapine or norclozapine may optionally be utilised in the form of a pharmaceutically acceptable salt and/or solvate and/or prodrug. In one embodiment of the invention clozapine or norclozapine is utilised in the form of a pharmaceutically acceptable salt. In a further embodiment of the invention clozapine or norclozapine is utilised in the form of a pharmaceutically acceptable solvate. In a further embodiment of the invention clozapine or norclozapine is not in the form of a salt or solvate. In a further embodiment of the invention clozapine or norclozapine is utilised in the form of a prodrug. In a further embodiment of the invention clozapine or norclozapine is not utilised in the form of a prodrug.

The term "pathogenic immunoglobulin driven B cell disease" includes B cell mediated disease, especially autoimmune disease, which involves pathogenic immunoglobulins (e.g. IgG, IgA and/or IgM) targeting a self-antigen (e.g. auto-antibody IgG, IgA and/or IgM) as a principal mechanism.

Suitably the pathogenic immunoglobulin driven B cell disease is a pathogenic IgG driven B cell disease. Alternatively, suitably it is a pathogenic IgA driven B cell disease.

The term "pathogenic IgG driven B cell disease" includes B cell mediated disease, especially autoimmune disease, which involves pathogenic IgG targeting a self-antigen (i.e. auto-antibody IgG) as a principal mechanism.

The term "pathogenic IgA driven B cell disease" includes B cell mediated disease, especially autoimmune disease, which involves pathogenic IgA targeting a self-antigen (i.e. auto-antibody IgA) as a principal mechanism.

The range of self-antigens involved in autoimmune diseases include desmoglein 3, BP180, BP230, (pemphigus), dystonin and/or type XVII collagen (pemphigoid), myelin (multiple sclerosis), pancreatic beta cell proteins (Type 1 diabetes mellitus), nicotinic acetylcholine receptors (myasthenia gravis), neuronal surface proteins (autoimmune epilepsy and encephalitis), 2-hydrolase (autoimmune Addison's disease), FcεRI (chronic autoimmune urticaria) and acetylcholine receptor (myasthenia gravis).

The range of self-antigens involved in pathogenic IgA driven B cell diseases include tissue transglutaminase (dermatitis herpetiformis and coeliac disease), gliadin IgA (coeliac disease) and dystonin and/or type XVII collagen (linear IgA disease).

Exemplary pathogenic IgG driven B cell diseases are autoimmune diseases including those which may be selected from the group consisting of the skin related diseases pemphigus vulgaris, pemphigus foliaceus, bullous pemphigoid, cicatricial pemphigoid, autoimmune alopecia, vitiligo, dermatitis herpetiformis and chronic autoimmune urticaria. Alternatively, the disease may be the gut related disease coeliac disease. Alternatively, the diseases may be selected from the group consisting of the thyroid gland related diseases Graves' disease and Hashimoto's thyroiditis. Alternatively, the diseases may be the pancreas related disease Type 1 diabetes mellitus. Alternatively, the disease may be the adrenal gland related disease autoimmune Addison's disease. Alternatively, the diseases may be selected from the group consisting of the haematological related diseases autoimmune haemolytic anaemia, autoimmune thrombocytopenic purpura and cryoglobulinemia. Alternatively, the disease may be the gut related disease pernicious anaemia. Alternatively, the diseases may be selected from the group consisting of the neurological related diseases myasthenia gravis, multiple sclerosis, neuromyelitis optica and autoimmune epilepsy and encephalitis. Alternatively, the diseases may be selected from the group consisting of the liver related diseases autoimmune hepatitis, primary biliary cirrhosis and primary sclerosing cholangitis.

Thus, in an embodiment, the invention provides (i) a compound selected from clozapine, norclozapine and prodrugs thereof and pharmaceutically acceptable salts and solvates thereof for use in the treatment or prevention of a pathogenic immunoglobulin driven B cell disease in a subject and (ii) a method of treatment or prevention of a pathogenic immunoglobulin driven B cell disease in a subject by administering to said subject an effective amount of a compound selected from clozapine, norclozapine and prodrugs thereof and pharmaceutically acceptable salts and solvates thereof wherein in the case of (i) and (ii) the pathogenic immunoglobulin driven B cell disease is a disease selected from the group consisting of pemphigus vulgaris, pemphigus foliaceus, bullous pemphigoid, cicatricial pemphigoid, autoimmune alopecia, vitiligo, dermatitis herpetiformis, chronic autoimmune urticaria, coeliac disease, Graves' disease, Hashimoto's thyroiditis, Type 1 diabetes mellitus, autoimmune Addison's disease, autoimmune haemolytic anaemia, autoimmune thrombocytopenic purpura, cryoglobulinemia, pernicious anaemia, myasthenia gravis, multiple sclerosis, neuromyelitis optica, autoimmune epilepsy and encephalitis, autoimmune hepatitis, primary biliary cirrhosis and primary sclerosing cholangitis.

Preferably the pathogenic IgG driven B cell disease is selected from pemphigus vulgaris, pemphigus foliaceus and bullous pemphigoid.

Exemplary pathogenic IgA driven B cell diseases may be selected from the group consisting of the skin related diseases dermatitis herpetiformis, linear IgA disease, pemphigus vulgaris, pemphigus foliaceus, cicatricial pemphigoid and bullous pemphigoid. Alternatively, the disease may be the gut related disease coeliac disease. Alternatively, the disease may be the kidney related disease IgA nephropathy.

Preferably the pathogenic IgA driven B cell disease is selected from dermatitis herpetiformis and linear IgA disease.

Clozapine is associated with high levels of CNS penetration which could prove to be a valuable property in treating some of these diseases (Michel. L. et al., 2015).

In certain diseases, more than one Ig type (such as IgG and IgA) may play a role in the pathology of the disease. For example, in dermatitis herpetiformis, coeliac disease, pemphigus vulgaris, pemphigus foliaceus, cicatricial pemphigoid and bullous pemphigoid, production of pathogenic IgA is thought to contribute towards the pathology as well as IgG.

In certain diseases, such as multiple sclerosis, vitiligo, Type 1 diabetes mellitus, autoimmune Addison's disease, dermatitis herpetiformis, coeliac disease, primary biliary cirrhosis, primary sclerosing cholangitis and autoimmune thrombocytopenic purpura there may also be a T cell component that contributes towards the pathology of the disease. This arises because B cells act as professional antigen-presenting cells for T cells (their importance is increased also due to their sheer numbers). B cells secrete significant amounts of cytokines that impact T cells. B-T interaction is involved in responses to T dependent protein antigens and class switching. Therefore, clozapine and norclozapine are expected to have an effect on T cells due to their effect on reducing B cell numbers.

Suitably the compound selected from clozapine, norclozapine and prodrugs thereof inhibits mature B cells, especially CSMBs and plasmablasts, particularly CSMBs. "Inhibit" means reduce the number and/or activity of said cells. Thus, suitably clozapine or norclozapine reduces the number of CSMBs and plasmablasts, particularly CSMBs.

In an embodiment the compound selected from clozapine, norclozapine and prodrugs thereof has the effect of decreasing CD19 (+) and/or CD19 (-) B-plasma cells.

The term "treatment" means the alleviation of disease or symptoms of disease. The term "prevention" means the prevention of disease or symptoms of disease. Treatment includes treatment alone or in conjunction with other therapies. Treatment embraces treatment leading to improvement of the disease or its symptoms or slowing of the rate of progression of the disease or its symptoms. Treatment includes prevention of relapse.

The term "effective amount" refers to an amount effective, at dosages and for periods of time necessary, to achieve the desired therapeutic result, in which any toxic or detrimental effects of the pharmacological agent are outweighed by the therapeutically beneficial effects. It is understood that the effective dosage will be dependent upon the age, sex, health, and weight of the recipient, kind of concurrent treatment, if any, frequency of treatment, and the nature of the effect desired. The most preferred dosage will be tailored to the individual subject, as is understood and determinable by one of skill in the art, without undue experimentation. Example dosages are discussed below.

As used herein, a "subject" is any mammal, including but not limited to humans, non-human primates, farm animals such as cattle, sheep, pigs, goats and horses; domestic animals such as cats, dogs, rabbits; laboratory animals such as mice, rats and guinea pigs that exhibit at least one symptom associated with a disease, have been diagnosed with a disease, or are at risk for developing a disease. The term does not denote a particular age or sex. Suitably the subject is a human subject.

It will be appreciated that for use in medicine the salts of clozapine and norclozapine should be pharmaceutically acceptable. Suitable pharmaceutically acceptable salts will be apparent to those skilled in the art. Pharmaceutically acceptable salts include those described by Berge, Bighley and Monkhouse J. Pharm. Sci. (1977) 66, pp 1-19. Such pharmaceutically acceptable salts include acid addition salts formed with inorganic acids e.g. hydrochloric, hydrobromic, sulphuric, nitric or phosphoric acid and organic acids e.g. succinic, maleic, acetic, fumaric, citric, tartaric, benzoic, p-toluenesulfonic, methanesulfonic or naphthalenesulfonic acid. Other salts e.g. oxalates or formates, may be used, for example in the isolation of clozapine and are included within the scope of this invention.

A compound selected from clozapine, norclozapine and prodrugs thereof and pharmaceutically acceptable salts and solvates thereof may be prepared in crystalline or non-crystalline form and, if crystalline, may optionally be solvated, e.g. as the hydrate. This invention includes within its scope stoichiometric solvates (e.g. hydrates) as well as compounds containing variable amounts of solvent (e.g. water).

A "prodrug", such as an N-acylated derivative (amide) (e.g. an N-acylated derivative of norclozapine) is a compound which upon administration to the recipient is capable of providing (directly or indirectly) clozapine or an active metabolite or residue thereof. Other such examples of suitable prodrugs include alkylated derivatives of norclozapine other than clozapine itself.

Isotopically-labelled compounds which are identical to clozapine or norclozapine but for the fact that one or more atoms are replaced by an atom having an atomic mass or mass number different from the atomic mass or mass number most commonly found in nature, or in which the proportion of an atom having an atomic mass or mass number found less commonly in nature has been increased (the latter concept being referred to as "isotopic enrichment") are also contemplated for the uses and method of the invention. Examples of isotopes that can be incorporated into clozapine or norclozapine include isotopes of hydrogen, carbon, nitrogen, oxygen, fluorine, iodine and chlorine such as ²H (deuterium), ³H, ¹¹C, ¹³C, ¹⁴C, ¹⁸F, ¹²³I or ¹²⁵I, which may be naturally occurring or non-naturally occurring isotopes.

Clozapine or norclozapine and pharmaceutically acceptable salts of clozapine or norclozapine that contain the aforementioned isotopes and/or other isotopes of other atoms are contemplated for use for the uses and method of the present invention. Isotopically labelled clozapine or norclozapine, for example clozapine or norclozapine into which radioactive isotopes such as ³H or ¹⁴C have been incorporated, are useful in drug and/or substrate tissue distribution assays. Tritiated, i.e. ³H, and carbon-14, i.e. ¹⁴C, isotopes are particularly preferred for their ease of preparation and detectability. ¹¹C and ¹⁸F isotopes are particularly useful in PET (positron emission tomography).

Since clozapine or norclozapine are intended for use in pharmaceutical compositions it will readily be understood that it is preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

In general, clozapine or norclozapine may be made according to the organic synthesis techniques known to those skilled in this field (as described in, for example, US3539573.

A compound selected from clozapine, norclozapine and prodrugs thereof and pharmaceutically acceptable salts and solvates thereof for use in therapy is usually administered as a pharmaceutical composition. Also provided is a pharmaceutical composition comprising clozapine or norclozapine, or a pharmaceutically acceptable salt and/or solvate and/or prodrug thereof and a pharmaceutically acceptable diluent or carrier. Said composition is provided for use in the treatment or prevention of a pathogenic immunoglobulin driven B cell disease in a subject wherein said compound causes mature B cells to be inhibited in said subject.

A compound selected from clozapine, norclozapine and prodrugs thereof and pharmaceutically acceptable salts and solvates thereof may be administered by any convenient method, e.g. by oral, parenteral, buccal, sublingual, nasal, rectal or transdermal administration, and the pharmaceutical compositions adapted accordingly. Other possible routes of administration include intratympanic and intracochlear. Suitably, a compound selected from clozapine, norclozapine and prodrugs thereof and pharmaceutically acceptable salts and solvates thereof are administered orally.

A compound selected from clozapine, norclozapine and prodrugs thereof and pharmaceutically acceptable salts and solvates thereof which are active when given orally can be formulated as liquids or solids, e.g. as syrups, suspensions, emulsions, tablets, capsules or lozenges.

A liquid formulation will generally consist of a suspension or solution of the active ingredient in a suitable liquid carrier(s) e.g. an aqueous solvent such as water, ethanol or glycerine, or a non-aqueous solvent, such as polyethylene glycol or an oil. The formulation may also contain a suspending agent, preservative, flavouring and/or colouring agent.

A composition in the form of a tablet can be prepared using any suitable pharmaceutical carrier(s) routinely used for preparing solid formulations, such as magnesium stearate, starch, lactose, sucrose and cellulose.

A composition in the form of a capsule can be prepared using routine encapsulation procedures, e.g. pellets containing the active ingredient can be prepared using standard carriers and then filled into a hard gelatin capsule; alternatively a dispersion or suspension can be prepared using any suitable pharmaceutical carrier(s), e.g. aqueous gums, celluloses, silicates or oils and the dispersion or suspension then filled into a soft gelatin capsule.

Typical parenteral compositions consist of a solution or suspension of the active ingredient in a sterile aqueous carrier or parenterally acceptable oil, e.g. polyethylene glycol, polyvinyl pyrrolidone, lecithin, arachis oil or sesame oil. Alternatively, the solution can be lyophilised and then reconstituted with a suitable solvent just prior to administration.

Compositions for nasal or pulmonary administration may conveniently be formulated as aerosols, sprays, drops, gels and powders. Aerosol formulations typically comprise a solution or fine suspension of the active ingredient in a pharmaceutically acceptable aqueous or non-aqueous solvent and are usually presented in single or multidose quantities in sterile form in a sealed container which can take the form of a cartridge or refill for use with an atomising device. Alternatively the sealed container may be a disposable dispensing device such as a single dose nasal or pulmonary inhaler or an aerosol dispenser fitted with a metering valve. Where the dosage form comprises an aerosol dispenser, it will contain a propellant which can be a compressed gas e.g. air, or an organic propellant such as a fluorochlorohydrocarbon or hydrofluorocarbon. Aerosol dosage forms can also take the form of pump-atomisers.

Compositions suitable for buccal or sublingual administration include tablets, lozenges and pastilles where the active ingredient is formulated with a carrier such as sugar and acacia, tragacanth, or gelatine and glycerine.

Compositions for rectal administration are conveniently in the form of suppositories containing a conventional suppository base such as cocoa butter.

Compositions suitable for topical administration to the skin include ointments, gels and patches.

In one embodiment the composition is in unit dose form such as a tablet, capsule or ampoule.

Compositions may be prepared with an immediate release profile upon administration (i.e. upon ingestion in the case of an oral composition) or with a sustained or delayed release profile upon administration.

For example, a composition intended to provide constant release of clozapine over 24 hours is described in WO2006/059194 the contents of which are herein incorporated in their entirety.

The composition may contain from 0.1% to 100% by weight, for example from 10 to 60% by weight, of the active material, depending on the method of administration. The composition may contain from 0% to 99% by weight, for example 40% to 90% by weight, of the carrier, depending on the method of administration. The composition may contain from 0.05mg to 1000mg, for example from 1.0mg to 500mg, of the active material (i.e. clozapine or norclozapine), depending on the method of administration. The composition may contain from 50 mg to 1000 mg, for example from 100mg to 400mg of the carrier, depending on the method of administration. The dose of clozapine or norclozapine used in the treatment or prevention of the aforementioned diseases will vary in the usual way with the seriousness of the diseases, the weight of the sufferer, and other similar factors. However, as a general guide suitable unit doses of clozapine as free base may be 0.05 to 1000 mg, more suitably 1.0 to 500mg, and such unit doses may be administered more than once a day, for example two or three a day. Such therapy may extend for a number of weeks or months.

A compound selected from clozapine, norclozapine and prodrugs thereof and pharmaceutically acceptable salts and solvates thereof may be administered in combination with another therapeutic agent for the treatment of pathogenic immunoglobulin driven B cell diseases (e.g. IgG or IgA driven B cell disease), such as those that inhibit B cells or B cell - T-cell interactions. Other therapeutic agents include for example: anti-TNFα agents (such as anti-TNFα antibodies e.g. infliximab or adalumumab), calcineurin inhibitors (such as tacrolimus or cyclosporine), antiproliferative agents (such as mycophenolate e.g. as mofetil or sodium, or azathioprine), general anti-inflammatories (such as hydroxychloroquine or NSAIDS such as ketoprofen and colchicine), mTOR inhibitors (such as sirolimus), steroids (such as prednisone), anti-CD80/CD86 agents (such as abatacept), anti-CD-20 agents (such as anti-CD-20 antibodies e.g. rituximab). anti- BAFF agents (such as anti- BAFF antibodies e.g. tabalumab or belimumab, or atacicept), immunosuppressants (such as methotrexate or cyclophosphamide), anti-FcRn agents (e.g. anti-FcRn antibodies) and other antibodies (such as ARGX-113, PRN-1008, SYNT-001, veltuzumab, ocrelizumab, ofatumumab, obinutuzumab, ublituximab, alemtuzumab, milatuzumab, epratuzumab and blinatumomab). Rituximab may be mentioned in particular.

Other therapies that may be used in combination with the invention include non-pharmacological therapies such as intravenous immunoglobulin therapy (IVIg), subcutaneous immunoglobulin therapy (SCIg) eg facilitated subcutaneous immunoglobulin therapy, plasmapheresis and immunoabsorption.

Thus the invention provides a compound selected from clozapine, norclozapine and prodrugs thereof and pharmaceutically acceptable salts and solvates thereof for use in the treatment or prevention of a pathogenic immunoglobulin driven B cell disease in combination with a second or further therapeutic agent for the treatment or prevention of a pathogenic immunoglobulin driven B cell disease (e.g. IgG or IgA driven B cell disease) e.g. a substance selected from the group consisting of anti-TNFα agents (such as anti-TNFα antibodies e.g. infliximab or adalumumab), calcineurin inhibitors (such as tacrolimus or cyclosporine), antiproliferative agents (such as mycophenolate e.g. as mofetil or sodium, or and azathioprine), general anti-inflammatories (such as hydroxychloroquine and NSAIDS such as ketoprofen and colchicine), mTOR inhibitors (such as sirolimus), steroids (such as prednisone), anti-CD80/CD86 agents (such as abatacept), anti-CD-20 agents (such as anti-CD-20 antibodies e.g. rituximab). anti- BAFF agents (such as anti- BAFF antibodies e.g. tabalumab or belimumab, or atacicept), immunosuppressants (such as methotrexate or cyclophosphamide), anti-FcRn agents (e.g. anti-FcRn antibodies) and other antibodies (such as ARGX-113, PRN-1008, SYNT-001, veltuzumab, ocrelizumab, ofatumumab, obinutuzumab, ublituximab, alemtuzumab, milatuzumab, epratuzumab and blinatumomab). Rituximab may be mentioned in particular.

When a compound selected from clozapine, norclozapine and prodrugs thereof and pharmaceutically acceptable salts and solvates thereof is used in combination with other therapeutic agents, the compounds may be administered separately, sequentially or simultaneously by any convenient route.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations. The individual components of combinations may also be administered separately, through the same or different routes. For example, a compound selected from clozapine, norclozapine and prodrugs thereof and pharmaceutically acceptable salts and solvates thereof and the other therapeutic agent may both be administered orally. Alternatively, a compound selected from clozapine, norclozapine and prodrugs thereof and pharmaceutically acceptable salts and solvates thereof may be administered orally and the other therapeutic agent via may be administered intravenously or subcutaneously.

Typically, a compound selected from clozapine, norclozapine and prodrugs thereof and pharmaceutically acceptable salts and solvates thereof is administered to a human.

### Examples

### Example 1

To assess a possible association between antibody deficiency and clozapine use the inventors undertook a cross-sectional case control study to compare the immunoglobulin levels and specific antibody levels (against Haemophilus B (Hib), Tetanus and Pneumococcus) in patients taking either clozapine or alternative antipsychotics.

### Method

Adults (>18yrs) receiving either clozapine or non-clozapine antipsychotics were recruited during routine clinic visits to ten Community Mental Health Trust (CMHT) outpatient clinics in Cardiff & Vale and Cwm Taf Health Boards by specialist research officers between November 2013 and December 2016 (Table 1). Following consent, participants completed a short lifestyle, drug history and infection questionnaire followed by blood sampling. Where required, drug histories were confirmed with the patient's General Practice records. Formal psychiatric diagnoses and antipsychotic medication use were confirmed using the medical notes, in line with other studies. Patients' admission rates were confirmed by electronic review for the 12-month period prior to recruitment. Patients with known possible causes of hypogammaglobulinemia including prior chemotherapy, carbamazepine, phenytoin, antimalarial agents, captopril, high-dose glucocorticoids, hematological malignancy and 22q11 deletion syndrome were excluded.

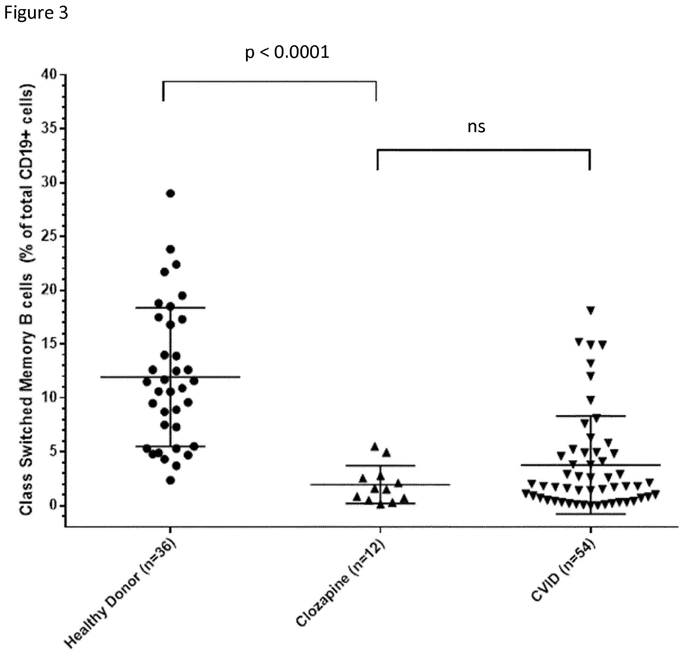

Clinical and immunological data from 13 patients taking clozapine, 11 of whom had been referred independently of the study for assessment in Immunology clinic, are presented in Table 3. Laboratory data on these, healthy controls and patients with common variable immunodeficiency (CVID) are shown in Figure 3. The 11 independently referred patients were excluded from the overall study analysis.

Immunoglobulin levels (IgG, IgA and IgM) were assayed by nephelometry (Siemens BN2 Nephelometer; Siemens), serum electrophoresis (Sebia Capillarys 2; Sebia, Norcross, GA, USA) and, where appropriate, serum immunofixation (Sebia Hydrasys; Sebia, Norcross, GA, USA). Specific antibody titres against Haemophilus influenzae, Tetanus and Pneumococcal capsular polysaccharide were determined by ELISA (The Binding Site, Birmingham, UK). Lymphocyte subsets, naïve T cells and EUROclass B cell phenotyping were enumerated using a Beckman Coulter FC500 (Beckman Coulter, California, USA) flow cytometer. All testing was performed in the United Kingdom Accreditation Service (UKAS) accredited Immunology Laboratory at the University Hospital of Wales. Laboratory adult reference ranges for immunoglobulin levels used were, IgG 6-16g/L, IgA 0.8-4g/L, IgM 0.5-2g/L.

Statistical analysis of the laboratory and clinical data was performed using Microsoft Excel and Graphpad Prism version 6.07 (Graphpad, San Diego, California, USA). Independent samples t-test were performed unless D'Agoustino & Pearson testing showed significant deviation from the Gaussian distribution, in which case the non-parametric Mann-Whitney test was used. All tests were two-tailed, using a significance level of p<0.05.

### Results

### Study Participants

A total of 291 patients taking clozapine and 280 clozapine-naïve patients were approached and 123 clozapine and 111 clozapine-naïve patients consented to the study (Table 1). Recruitment was stopped as per protocol when the target of 100 patients in each group had been achieved. There were small differences in gender with more males in the clozapine-treated group (53% versus 50%) and a lower mean age in the clozapine group (45 versus 50 years). These differences are unlikely to be relevant as there are no gender differences in the adult reference range for serum immunoglobulins and there is a male predominance in schizophrenia. Levels of smoking, diabetes, COPD/asthma, and alcohol intake were similar between the groups. More patients were admitted to hospital with infection in the clozapine group (0.12 vs 0.06 per patient year) and more took >5 courses of antibiotics per year compared with controls (5.3% vs 2%). The possible impact of a diagnosis of schizophrenia, medications and smoking as risk factors for antibody deficiency were assessed in a subgroup analysis (Table 2).

**Table 1 Clozapine-treated and clozapine-naïve patient characteristics**

| | **Clozapine-Treated** | **Clozapine-Naive** |
|---|---|---|
| **Total screened** | 291 | 280 |
| **Declined, lacked capacity, or unable to obtain blood sample** | 168 | 169 |
| **Initial Screening Sex (M : F)** | 123 (81:42) | 111 (56:55) |
| **Mean age, years (Range)** | 45.3 (22.0 - 78.0) | 50.3 (21.6 - 78.0) |
| | | |
| **Post-exclusion (% total screened)** | 94 (32%) | 98 (35%) |
| **Sex (M : F)** | 64:30 | 54:44 |
| **Mean age, years (Range)** | 44.4 (22.0-78.0) | 50.4 (21.6-78.0) |
| | | |
| **Primary Psychiatric Diagnosis** | | |
| • **Schizophrenia** | 87 | 58 |
| • **Schizoaffective** | 1 | 5 |
| • **Bipolar** | 0 | 11 |
| • **Psychosis** | 0 | 15 |
| • **Depression** | 0 | 3 |
| • **Personality Disorder** | 2 | 2 |
| • **Anxiety disorder** | 0 | 2 |
| • **Electronic record incomplete** | 4 | 2 |
| | | |
| **Dual antipsychotic treatment** | 30.9% | 11.2% |
| **Duration antipsychotic use (median, range), years** | 8.0 (0.1-20) | 7.0 (0.1-44) |
| | | |
| **Current smoking (%)** | 60.6% | 56.1% |
| **Diabetes (%)** | 20.2% | 17.3% |
| **COPD/Asthma (%)** | 13.8% | 16.3% |
| **Alcohol intake mean (units/week), range** | 5.3 (0-60) | 6.0 (0-68) |
| **Antibiotic courses per year** | | |
| **• Nil courses** | 61.7% | 63.3% |
| **• 1-5 courses** | 33.0% | 34.7% |
| **• >5 courses** | 5.3% | 2.0% |
| **Admission frequency in 12-month period** | | |
| **All cause** | 21 (14 patients) | 14 (13 patients) |
| **Infection-related** | 15 (10 patients) | 7 (6 patients) |

### Effects of clozapine on antibody levels

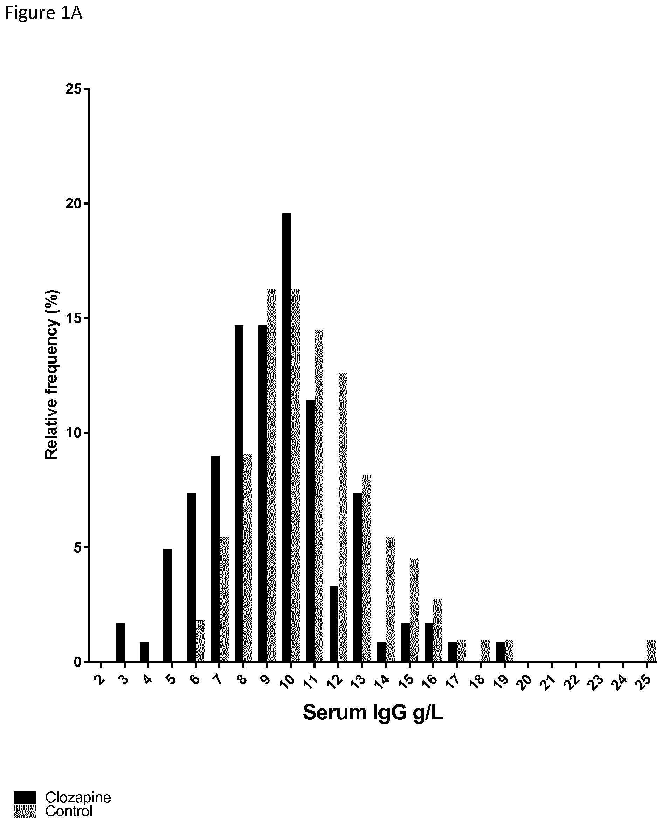

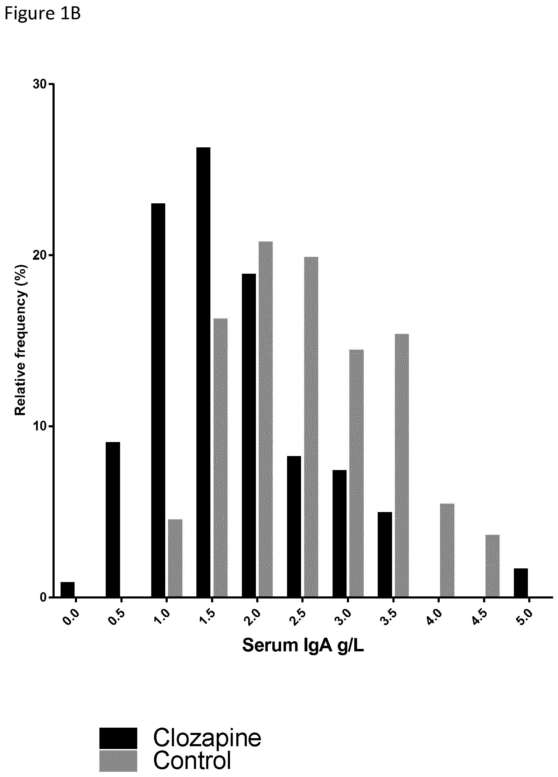

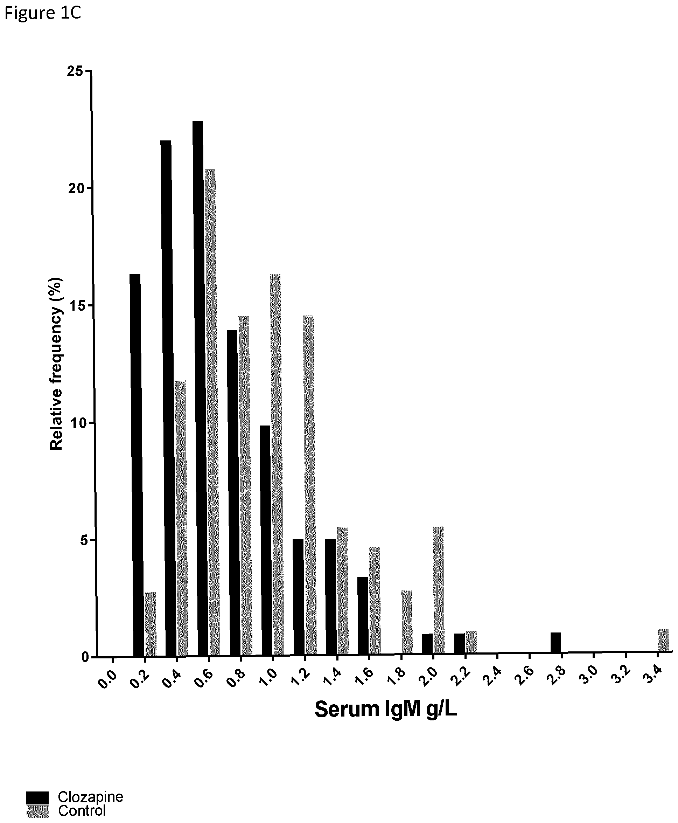

Figure 1 shows significantly reduced concentrations of all three immunoglobulin classes (IgG, IgA and IgM) in patients receiving clozapine, with a shift towards lower immunoglobulin levels in the distribution as a whole for each of IgG, IgA and IgM compared to the clozapine-naïve control group. The percentages of the 123 patients having immunoglobulin levels below the reference range were IgG 9.8% (p<0.0001), IgA 13.0% (p<0.0001) and IgM 38.2% (p<0.0001) compared with the 111 clozapine-naïve IgG 1.8%, IgA 0.0% and IgM 14.4%. Large percentages of both clozapine-treated and clozapine-naïve patients had specific antibody levels below the protective levels for HiB (51% and 56% less than 1 mcg/ml, (Orange JS., 2012)), Pneumococcus (54% and 56% less than 50mg/L, (Chua I., 2011)) and Tetanus (12% and 14% less than 0.1IU/ml). The Pneumococcal IgA (31U/ml vs 58.4U/ml p< 0.001) and IgM (58.5U/ml vs 85.0U/ml p<0.001) levels are significantly lower in clozapine-treated versus clozapine-naïve patients.

Subgroup analysis (Table 2) was undertaken to determine if the reductions in immunoglobulins were potentially explained by confounding factors including any other drugs, a diagnosis of schizophrenia and smoking. The assessment of the effect of excluding other secondary causes of antibody deficiency (plus small numbers where additional diagnoses were uncovered - Table 1) is shown in Column B. The number of patients excluded on the basis of taking anti-epileptic medications was higher in the clozapine-treated group and is likely to reflect the use of these agents for their mood stabilizing properties rather than as treatment for epilepsy.

**Table 2 Immunoglobulin levels and specific antibody levels in sub-groups A-D**

| | **A** | | **B** | | **C** | | **D** | |
|---|---|---|---|---|---|---|---|---|
| **Medication:** | **Clozapine** | **Control** | **Clozapine** | **Control** | **Clozapine** | **Control** | **Clozapine** | **Control** |
| **Diagnosis:** | All | All | All | All | Schizophrenia diagnoses only | | All | All |
| **Smoking:** | All | All | All | All | All | All | Smoke | rs only |
| **Possible secondary causes excluded** | No | No | Yes | | Yes | | Yes | |
| **Sample size:** | **123** | **111** | **94** | **98** | **87** | **58** | **57** | **55** |
| **Serum IgG (Reference range 6-16g/L)** | **95% CI:** 0.89 - **2.32 ****** | | **95% CI: 0.98 to 2.59 ****** | | **95% CI: 0.92 to 2.77 ***** | | **Non-Gaussian distribution** † | |
| **< 3** | 0.8% | 0.0% | 1.1% | 0.0% | 1.2% | 0.0% | 1.8% | 0.0% |
| **< 4** | 1.6% | 0.0% | 1.1% | 0.0% | 1.2% | 0.0% | 1.8% | 0.0% |
| **< 5** | 3.3% | 0.0% | 2.1% | 0.0% | 2.3% | 0.0% | 1.8% | 0.0% |
| **< 6** | 9.8% | 1.8% | 8.4% | 1.0% | 9.2% | 1.7% | 8.8% | 1.8% |
| | | | | | | | | |
| **Serum IgA (Reference range 0.8- 4.0 g/L)** | **95% CI: 0.55 to 1.01 ****** | | **95% CI: 0.55 to 1.05 ****** | | **95% CI: 0.59 to 1.19 ****** | | **95% CI: 0.41 to 1.04 ****** | |
| **< 0.5** | 1.6% | 0.0% | 2.1% | 0.0% | 2.3% | 0.0% | 3.5% | 0.0% |
| **< 0.6** | 2.4% | 0.0% | 2.1% | 0.0% | 3.5% | 00% | 3.5% | 0.0% |
| **< 0.7** | 6.5% | 0.0% | 6.4% | 0.0% | 6.9% | 0.0% | 3.5% | 0.0% |
| **< 0.8** | 13.0% | 0.0% | 13.8% | 0.0% | 14.9% | 0.0% | 10.5% | 0.0% |
| | | | | | | | | |
| **Serum IgM (Reference range 0.5 - 1.9 g/L)** | **Non-Gaussian distribution ††††** | | **95% CI: 0.10 to** 0.38 ******* | | **95% CI: 0.06** to 0.38 ** | | 95% **CI:** 0.02 to 0.39 ***** | |
| **< 0.2** | 8.1% | 0.0% | 5.3% | 0.0% | 5.8% | 0.0% | 1.78% | 0.0% |
| **< 0.3** | 16.3% | 2.7% | 12.8% | 3.1% | 12.6% | 5.2% | 12.3% | 1.8% |
| **< 0.4** | 29.3% | 8.1% | 26.6% | 8.2% | 27.6% | 6.9% | 26.3% | 9.1% |
| **< 0.5** | 38.2% | 14.4% | 34.0% | 15.3% | 35.6% | 13.8% | 33.3% | 18.2% |
| | | | | | | | | |
| **IgG-Pneumococcus (mg/L)** | **95% CI: -8.25 to 21.92 (ns)** | | **95% CI: -11.21 to 22.63 (ns)** | | **95% CI: -20.50 to 17.54 (ns)** | | **95% CI: -23.64 to 21.70 (ns)** | |
| **< 35** | 39.0% | 43.2% | 38.3% | 40.8% | 37.9% | 43.1% | 45.6% | 43.6% |
| **< 50** | 53.7% | 55.9% | 52.1% | 54.1% | 50.6% | 60.3% | 54.4% | 63.6% |
| | | | | | | | | |
| **IgG-Tetanus (IU/ml)** | Non-Gaussian distribution (ns) | | Non-Gaussian distribution (ns) | | Non-Gaussian distribution (ns) | | Non-Gaussian distribution (ns) | |
| **< 0.1** | 12.2% | 13.5% | 10.6% | 13.3% | 11.5% | 13.8% | 12.3% | 14.6% |
| | | | | | | | | |
| **IgG-Haemophilus B (mcg/ml)** | **Non-Gaussian distribution (ns)** | | **Non-Gaussian distribution (ns)** | | **Non-Gaussian distribution (ns)** | | **Non-Gaussian distribution (ns)** | |
| **< 1.0** | 51.2% | 55.9% | 51.1% | 54.1% | 49.4% | 53.5% | 50.9% | 60.0% |
| | | | | | | | | |
| **Sample size:** | **118** | **85** | **89** | **77** | **84** | **45** | **54** | **45** |
| **IgA-Pneumococcus (U/)** | 31 ± 3.97 ******* | 58.4 ± 6.7 | 30.8 ± 4.7 †††† | 58.8 ± 7.0 | 31.6 ± 4.9 ††† | 49.9 ± 7.6 | 30.7 ± 5.7 †††† | 61.3 ± 9.5 |
| **IgM-Pneumococcus (U/L)** | 58.5 ± 4.2 ******* | 85 ± 6.9 | 59.8 ± 4.9 ** | 85.8 ± 7.4 | 60.4 ± 5.1 * | 78.6 ± 7.1 | 61.6 ± 7.0 tt | 91.7 ± 10.3 |

| | | | | | | | | |
|---|---|---|---|---|---|---|---|---|
| Data shown as mean ± 1 SEM unless otherwise stated. * **Independent T test (normally distributed) or † Mann-Whitney (non-normally distributed)** **Levels of significance: */† p<0.05, **/†† p<0.005, ***/††† p<0.0005, ****/†††† p<0.0001** | | | | | | | | |

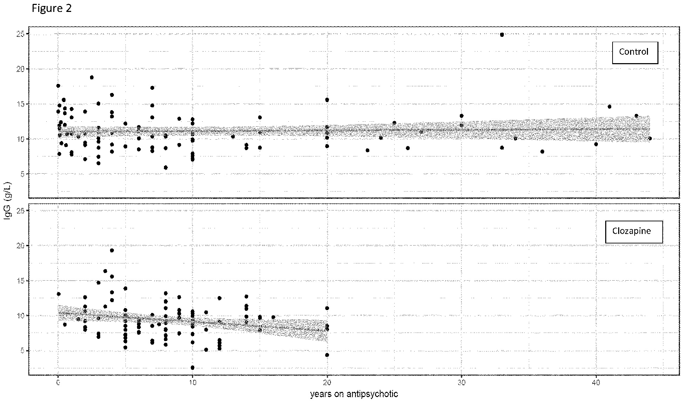

The association of clozapine with reduced IgG, IgA, IgM and Pneumococcal IgA and IgM remained statistically significant in all subgroups with 95% confidence intervals including when psychiatric diagnoses were restricted to schizophrenia only (Column C), and when non-smokers were excluded (Column D). When secondary causes of antibody deficiency were excluded (Column B) the odds ratios (with 95% confidence interval) for reduced immunoglobulins were IgG 9.02 (1.11- 73.7), IgA: 32.6 (1.91- 558) and IgM: 2.86 (1.42 - 5.73). In addition, a longer duration of clozapine therapy is associated with lower serum IgG levels (p 0.014) shown in Figure 2. This is not observed in clozapine-naïve patients treated with alternative antipsychotic drugs, despite a longer treatment duration than the clozapine therapy group.

### Immunological assessment of referred patients taking clozapine

Thirteen patients on clozapine were independently referred for assessment of antibody deficiency to Immunology clinic. Two had previously been recruited to the study and the eleven others are not included in the study to avoid bias. Five of the thirteen patients had been identified through the all Wales calculated globulin screening program. It was thus possible to undertake a more detailed immunological assessment in this group of thirteen 'real life' patients to provide additional background information (Table 3).

**Table 3 Immunological characteristics of the 13 referred clozapine patients**

| **Referral Reason** | **Age** | **Smoking** | **Relevant Medication** | **Clozapine duration** | **CSMB (6.5-29.1%)** | | **Intervention** | **Follow-up /months** |
|---|---|---|---|---|---|---|---|---|
| Recurrent respiratory tract infection (12 per year). | 47 | 20 pack years | Clozapine 250mg Sodium Valproate 1g Risperidone | > 4 | IgG <1.34 | 0.3 % | Prophylactic antibiotics Failure to respond to haemophilus and pneumococcal vaccination. Commenced SCI_{g} 9.6g weekly in nursing home. Recently discontinued clozapine due to neutropenia. | 120 |
| | | | | | IgA <0.22 | | | |
| | | | | | IgM <0.17 | | | |
| Low calculated globulin **Included in study** | 46 | 42 pack years | Clozapine 575mg Senna, fibrogel, cyclizine | 15 | IgG 5.24 | 2.77 % | Prompt antibiotic therapy Durable pneumococcal vaccine response Continues clozapine | 69 |
| | | | | | IgA 0.49 | | | |
| | | | | | IgM 0.41 | | | |
| Low calculated globulin. | 51 | 34 pack years | Clozapine 200mg Amisulpride | 5 | IgG 2.68 | 5.50 % | Prophylactic antibiotics Failure to responds to haemophilus and pneumococcal vaccination. Continues clozapine, Considering immunoglobulin replacement | 48 |
| | | | | | IgA 0.38 | | | |
| | | | | | IgM <0.17 | | | |
| Persistent cough for over a year and remains productive of green sputum despite several courses of **antibiotics.** | 63 | 60 pack years | Clozapine 400mg Olanzapine Trihexyphenid yl | 7.5 | IgG 2.98 | 0.5 % | Prophylactic antibiotics | 42 |
| | | | | | IgA <0.22 | | Non-durable pneumococcal vaccine response Commenced IVIg 40g 3 weekly Clozapine stopped with resultant psychotic episode. Clozapine restarted with GCSF cover Continues on SCIg and clozapine. | |
| | | | | | IgM 0.23 | | | |
| Recurrent respiratory infections | 49 | 55 pack years | Clozapine 300mg Sodium Valproate, | 7 | IgG 1.2 | 0.14 % | Prophylactic antibiotics Failure to respond to pneumococcal vaccination. IVIg 40g 3 weekly Continues clozapine | 32 |
| | | | | | IgA undetectable | | | |
| | | | | | IgM 0.07 | | | |
| Low calculated globulins | | | Pirenzapine, aripiprazole | | | | | |
| Recurrent chest infections Low calculated globulin | 63 | 20 pack years, stopp ed 30 years ago | Clozapine 250mg - **stopped** Lithium 400mg Levothyroxine Calchichew Citalopram | 10 years Stopped 24 months ago | IgG 3.3 | 1.58 % | Prophylactic azithromycin : 4 chest infections in 3 months Failure to respond to pneumococcal vaccination Clozapine stopped- red flags with neutropenia IgG rose to 5.95 from 3.3g/L, IgA 0.29, IgM 0.49 after 24 months CSMB rose to 2.77% | 24 |
| | | | | | IgA 0.26 | | | |
| | | | | | IgM 0.41 | | | |
| 7 courses of antibiotics for chest infections past 12 months, 9 GP visits No clozapine red-flags **Included in study** | 59 | 47 pack years | Clozapine 450mg Omeprazole, pirenzapine, venlafaxine, metformin, saxagliptin, atorvastatin | 10 | IgG 2.38 | 2.54 % | Prophylactic antibiotics Failure to respond to pneumococcal vaccination. Commenced IVIg 30g 3-weekly Continues clozapine | 15 |
| | | | | | IgA <0.22 | | | |
| | | | | | IgM <0.17 | | | |
| Recurrent respiratory infections | 46 | 74 pack years | Clozapine 450mg Sertaline, montelukast, simvastatin, seretide, salbulatamol, temazepam | 21 | IgG 4.24 | 0.84 % | Prophylactic antibiotics Failure to respond to pneumococcal vaccination. Commenced SCIg Continues clozapine | 12 |
| | | | | | IgA <0.22 | | | |
| | | | | | IgM <0.17 | | | |
| Recurrent respiratory tract infections | 50 | 60 pack years | Clozapine 700mg Amisulpride, cholecalcifero I, cod liver oil | >7 | IgG 6.65 | 4.95 % | Prophylactic antibiotics Failure to responds to haemophilus and pneumococcal vaccination. Continues clozapine | 12 |
| | | | | | IgA <0.22 | | | |
| | | | | | IgM <0.17 | | | |
| Low calculated globulin | 51 | 12 pack years | Clozapine 575mg Fibrogel, lactulose, cod liver oil, citalopram | 11 | IgG 5.61 | 2.10 % | Prompt antibiotic therapy Failure to respond to pneumococcal vaccination. Continues clozapine | 6 |
| | | | | | IgA 0.81 | | | |
| | | | | | IgM 0.18 | | | |
| Recurrent skin infections | 61 | 15/da y | Clozapine 325mg Sodium valproate, metformin, exenatide, ciitalopram, | >4 | IgG 4.79 | 1.49 % | Prompt antibiotics Assessment of vaccine responses ongoing Continues clozapine | 6 |
| | | | | | IgA 0.63 | | | |
| | | | | | IgM <0.17 | | | |
| | | | Fultium D3, Omeprazole, | | | | | |
| Calculated globulin | 36 | 35 pack years | Clozapine - **stopped** 2 years prior to referral Procyclidine, folic acid, diazepam, paracetamol | Stopped | IgG 4.8 | N/A | Declined further blood tests | 5 |
| | | | | | IgA 0.54 | | | |
| | | | | | IgM 0.3 | | | |
| Recurrent respiratory tract infections. Clozapine-induced sialorrhoea. | 57 | 20-40 pack years | Clozapine 750mg Amisulpride | > 4 | IgG <1.34 | 0.3-0.7 % | Prophylactic antibiotics Failure to respond to pneumococcal vaccination IVIg 40g every 3 weekly Stopped clozapine during chemotherapy | 42 |
| | | | | | IgA <0.22 | | | |
| | | | | | IgM <0.17 | | | |

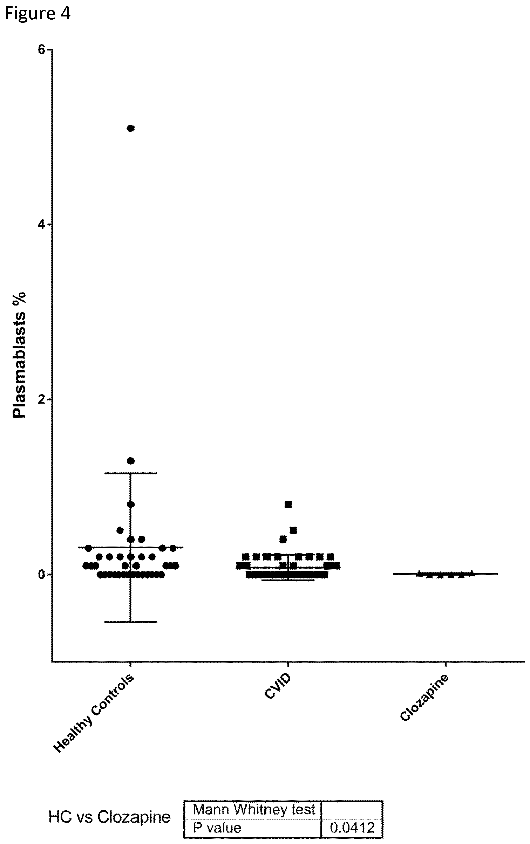

Immunoglobulins were reduced in all patients (mean IgG 3.6g/L, IgA 0.34g/L and IgM 0.21g/L). There was no severe overall lymphopenia or B cell lymphopenia, however, all patients had a major reduction in the percentage of CSMB (mean 1.87%, reference range 6.5-29.1%). A substantial reduction of CSMB is characteristic of patients with common variable immunodeficiency (CVID), the commonest severe primary immunodeficiency in adults. The percentages of CSMB in these clozapine-treated and CVID patients compared to healthy controls are shown in Figure 3 (p<0.0001), The plasmablast levels for 6 of the clozapine subjects compared to CVID patients and healthy controls are shown in Figure 4 (p=0.04). A reduction of plasmablasts is also characteristic of patients with common variable immunodeficiency (CVID) and this was also observed in clozapine treated patients. Responses to vaccination were impaired in 10/11 patients assessed and management included emergency backup antibiotics for 2/13 patients, prophylactic antibiotics in 9/13 and 6/13 patients were treated with immunoglobulin replacement therapy (IGRT). No patients discontinued clozapine because of antibody deficiency. The inflammatory or granulomatous complications which occur in a subset of CVID patients were not observed.

One patient subsequently discontinued clozapine because of neutropenia which normalized on clozapine cessation. Over the following 24 months the serum IgG level gradually increased from 3.3g/L to 4.8g/L and then 5.95g/L while IgA and IgM remained low. The increase in IgG was accompanied by a concomitant increase in class switched memory B cells from 1.58 - 2.77%, suggesting a gradual recovery on withdrawal of clozapine.

### Summary of Data

Clozapine treatment in patients led to a significant reduction of all immunoglobulin types. Percentages of patients below the immunoglobulin reference ranges were higher in clozapine treated (n=123) as compared with clozapine naive patients (n=111) (IgG <6g/L: 9.8% vs 1.8%; IgA <0.8g/L: 13.1% vs 0..0%; IgM <0.5g/L: 38.2% vs 14.2%) (p<0.0001) (see Figure 1)

Extending the duration of clozapine treatment was associated with progressively reduced IgG levels in patients treated with clozapine but not in clozapine naive patients who were on other antipsychotic medication (see Figure 2).

Specific IgG antibodies were below protective levels in both clozapine-treated and clozapine-naïve groups (HiB 51.2% vs 55.9%; Pneumococcal 53.7% vs 55.9%; Tetanus 12.2% vs 13.5%)). However, pneumococcal IgA and IgM levels were significantly lower in clozapine-treated patients as compared with clozapine-naïve patients (IgA 31.0 U/L vs 58.4 U/L; IgM 58.5 U/L vs 85 U/L) (p<0.001) (see Table 2).

Mean levels of CSMBs were significantly reduced at 1.87% in clozapine-treated patients referred independently to clinic and not included in the overall study (n=12) and in CVID patients (n=54) as compared with healthy controls (n=36) and the reference range of 6.5-29.1% (p<0.0001) (see Figure 3). Mean levels of plasmablasts were also reduced in clozapine-treated patients (p=0.04).

Thus, clozapine appears to have profound influence on the pathways involved in B cell maturation and pathogenic antibody (particularly pathogenic IgG and IgA antibody) production and thus is expected to be useful in treating pathogenic limmunoglobulin driven B cell mediated diseases.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.
All patents and patent applications referred to herein are incorporated by reference in their entirety.

### References

Chua I. et al., J Allergy Clin Immunol., 2011;127:1410-1
Guitton C. et al., Clin. Pharma., 1999;39(7):721-728
Hinze-Selch et al., Neuropsychopharmacology, 1998;19(2):114-122
Hung GC. et al., J Clin Psychiatry, 2016;77:60-6
Jolles et al., Clin. Exp. Immuno., 2014;177:671-678
Kappos L. et al., Lancet Neurol, 2014; 13:353-63
Kuo CJ. et al., Schizophr Bull., 2013;39:648-57
Leung JG. et al., 3rd Psychosomatics, 2017;58(2):164-172
Leung JG. et al., 3rd. Characterization of Admission Types in Medically Hospitalized Patients Prescribed Clozapine. Psychosomatics 2016.
Lozano et al., Ther. Adv. Psychopharmacol., 2016, 61(1):58-60
Michel L. et al., Front. Immunol., 24 December 2015; B Cells in the Multiple Sclerosis Central Nervous
System: Trafficking and Contribution to CNS-Compartmentalized Inflammation
Orange JS. et al., J Allergy Clin Immunol., 2012;130:S1-24
Richez C. et al., Expert Opin Invest Drugs 2014; 23:1285-94
Stoeker ZR. et al., Int. Clin. Psychopharmacol. 2017;32(3):155-160
Stohl W et al., Arthritis Rheum., 2012; 64:2328-37

## Claims

1. A compound selected from clozapine, norclozapine and prodrugs thereof and pharmaceutically acceptable salts and solvates thereof for use in the treatment or prevention of a pathogenic immunoglobulin driven B cell disease in a subject wherein said compound causes mature B cells to be inhibited in said subject.

2. The compound for use according to claim 1 wherein the compound is clozapine.

3. The compound for use according to claim 1 or claim 2 wherein the mature B cells are class switched memory B cells.

4. The compound for use according to claim 1 or claim 2 wherein the mature B cells are plasmablasts.

5. The compound for use according to any one of claims 1 to 4 wherein the pathogenic immunoglobulin driven B cell disease is a pathogenic IgG driven B cell disease.

6. The compound for use according to any one of claims 1 to 5 wherein the pathogenic immunoglobulin driven B cell disease is a disease selected from the group consisting of pemphigus vulgaris, pemphigus foliaceus, bullous pemphigoid, cicatricial pemphigoid, autoimmune alopecia, vitiligo, dermatitis herpetiformis, chronic autoimmune urticaria, coeliac disease, Graves' disease, Hashimoto's thyroiditis, Type 1 diabetes mellitus, autoimmune Addison's disease, autoimmune haemolytic anaemia, autoimmune thrombocytopenic purpura, cryoglobulinemia, pernicious anaemia, myasthenia gravis, multiple sclerosis, neuromyelitis optica, autoimmune epilepsy and encephalitis, autoimmune hepatitis, primary biliary cirrhosis and primary sclerosing cholangitis.

7. The compound for use according to claim 6 wherein the pathogenic immunoglobulin driven B cell disease is a disease selected from the group consisting of pemphigus vulgaris, pemphigus foliaceus and bullous pemphigoid.

8. The compound for use according to any one of claims 1 to 4 wherein the pathogenic immunoglobulin driven B cell disease is a pathogenic IgA driven B cell disease.

9. The compound for use according to any one of claims 1 to 5 and 8 wherein the pathogenic immunoglobulin driven B cell disease is a disease selected from the group consisting of dermatitis herpetiformis, linear IgA disease, coeliac disease, IgA nephropathy, pemphigus vulgaris, pemphigus foliaceus, cicatricial pemphigoid and bullous pemphigoid.

10. The compound for use according to claim 8 wherein the pathogenic immunoglobulin driven B cell disease is a disease selected from the group consisting of dermatitis herpetiformis and linear IgA disease.

11. A pharmaceutical composition comprising a compound selected from clozapine, norclozapine and prodrugs thereof and pharmaceutically acceptable salts and solvates thereof; and a pharmaceutically acceptable diluent or carrier, for use in the treatment or prevention of a pathogenic immunoglobulin driven B cell disease in a subject wherein said compound causes mature B cells to be inhibited in said subject.

12. The pharmaceutical composition for use according to claim 11 wherein the pharmaceutical composition is administered orally.

13. The pharmaceutical composition for use according to claims 11 or claim 12 wherein the mature B cells are class switched memory B cells or plasmablasts.

14. A compound selected from clozapine, norclozapine and prodrugs thereof and pharmaceutically acceptable salts and solvates thereof for use according to any one of claims 1 to 10 in combination with a second or further therapeutic agent for the treatment or prevention of a pathogenic immunoglobulin driven B cell disease.

15. The compound selected from clozapine, norclozapine and prodrugs thereof and pharmaceutically acceptable salts and solvates thereof for use according to claim 14 wherein the second or further substance for the treatment or prevention of a pathogenic immunoglobulin driven B cell disease is selected from anti-TNFα agents (such as anti-TNFα antibodies e.g. infliximab or adalumumab), calcineurin inhibitors (such as tacrolimus or cyclosporine), antiproliferative agents (such as mycophenolate e.g. as mofetil or sodium, or azathioprine), general anti-inflammatories (such as hydroxychloroquine or NSAIDS such as ketoprofen and colchicine), mTOR inhibitors (such as sirolimus), steroids (such as prednisone), anti-CD80/CD86 agents (such as abatacept), anti-CD-20 agents (such as anti-CD-20 antibodies e.g. rituximab). anti- BAFF agents (such as anti- BAFF antibodies e.g. tabalumab or belimumab, or atacicept), immunosuppressants (such as methotrexate or cyclophosphamide), anti-FcRn agents (e.g. anti-FcRn antibodies) and other antibodies (such as ARGX-113, PRN-1008, SYNT-001, veltuzumab, ocrelizumab, ofatumumab, obinutuzumab, ublituximab, alemtuzumab, milatuzumab, epratuzumab and blinatumomab).
